# EUROPÄISCHE PATENTSCHRIFT

(11) **EP 0 344 776 B1**
(45) Veröffentlichungstag und Bekanntmachung des Hinweises auf die Patenterteilung: **21.09.1994**
(21) Anmeldenummer: 89109936.8
(22) Anmeldetag: 01.06.1989
(51) Int. Cl.: C07D 307/79, C07D 307/82, D06L 3/12, C07C 309/29

(54) **Benzofuranylbiphenyle**
Benzofuranyl biphenyls
Benzofuranylbiphényls

(30) Priorität: 02.06.1988 CH 2090/88
(43) Veröffentlichungstag der Anmeldung: 06.12.1989
(73) Patentinhaber: CIBA-GEIGY AG, 4002 Basel (CH)
(72) Erfinder: Weber, Kurt, Dr., CH-4052 Basel (CH); Meyer, Hans Rudolf, Dr., CH-4102 Binningen (CH); Kaschig, Jürgen, Dr., D-7800 Freiburg (DE); Eckhardt, Claude, Dr., F-68400 Riedisheim (FR)
(74) Vertreter: TER MEER STEINMEISTER & PARTNER GbR

(56) Entgegenhaltungen:
- DE-A- 2 711 363
- DE-A- 2 843 850
- US-A- 4 002 423

## Beschreibung

Die vorliegende Anmeldung betrifft sulfonierte Benzofuranylbiphenylverbindungen sowie deren Herstellung und Verwendung als optische Aufheller.

Gemische von sulfonierten Benzofuranylbiphenylverbindungen mit undefinierter Zusammensetzung und Struktur sowie deren Verwendung als optische Aufheller sind lange bekannt (DE-A-22 38 734, DE-A-22 38 628, DE-A-23 61 338 und DE-A-28 43 850). Jedoch war es bislang nicht möglich, Einzelverbindungen mit einheitlichen Strukturen herzustellen.

Es hat sich nun überraschenderweise gezeigt, daß sulfonierte und strukturell definierte Benzofuranylbiphenylverbindungen nach bestimmten Verfahren gezielt herstellbar sind und daß diese Einzelverbindungen hervorragende Aufhelleigenschaften, z. B. zum Aufhellen von Textilien und Papier, besitzen.

Gegenstand der Anmeldung sind somit Benzofuranylbiphenylverbindungen der Formel (I) 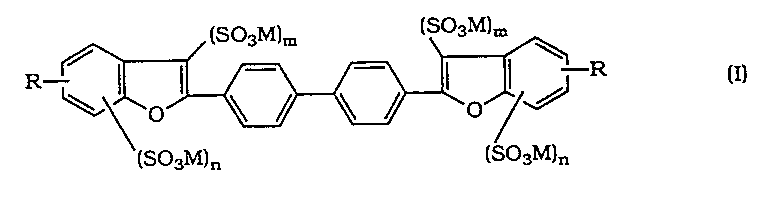
die gegebenenfalls mehrfach mit Resten R = Wasserstoff, C₁-C₄-Alkyl, C₁-C₄-Alkoxy, Halogen, Phenoxy und Benzyloxy substituiert sind und worin M = Wasserstoff und/oder ein Äquivalent eines nicht-chromophoren Kations und n eine Zahl Null oder 1 und m eine Zahl Null oder 1 bedeuten, mit der Bedingung, daß n und m nicht beide Null oder 1 sind.

M in der Bedeutung eines nicht-chromophoren Kations steht beispielsweise für Erdalkalimetall wie Magnesium und Calcium, vorzugsweise jedoch für Alkalimetall wie Lithium, Natrium, Kalium sowie gegebenenfalls substituiertes Ammonium wie Ammonium, Mono-, Di- oder Triethanolammonium, Mono-,Di- oder Tri-propanolammonium oder Tri- oder Tetramethylammonium.

Bevorzugt sind Verbindungen der Formel (II) und (IV) 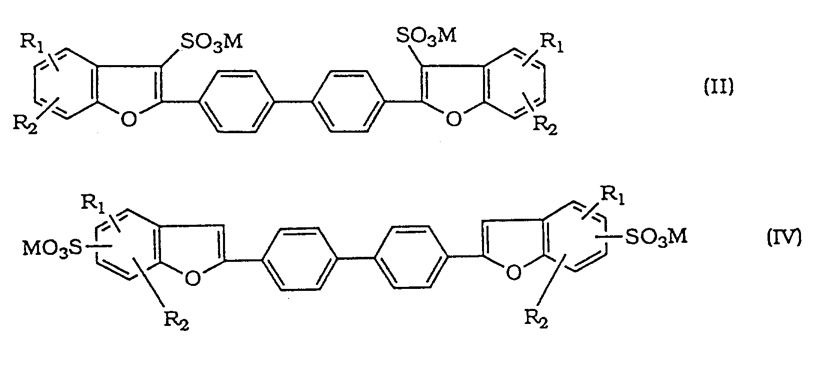
worin
R₁ = Wasserstoff, C₁-C₄-Alkyl, Chlor, C₁-C₄-Alkoxy, Phenoxy oder Benzyloxy, R₂ = Wasserstoff, C₁-C₄-Alkyl, Chlor oder C₁-C₄-Alkoxy und M = Wasserstoff und/oder ein Äquivalent eines nicht chromophoren Kations bedeuten und insbesondere Verbindungen der Formel (V) 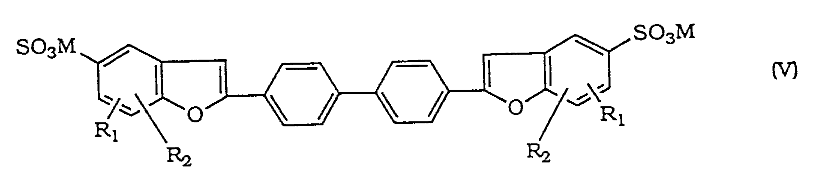
worin R₁, R₂ und M die oben angegebene Bedeutung haben.

Von besonderem Interesse sind jedoch Verbindungen der Formel (III) und (VI) 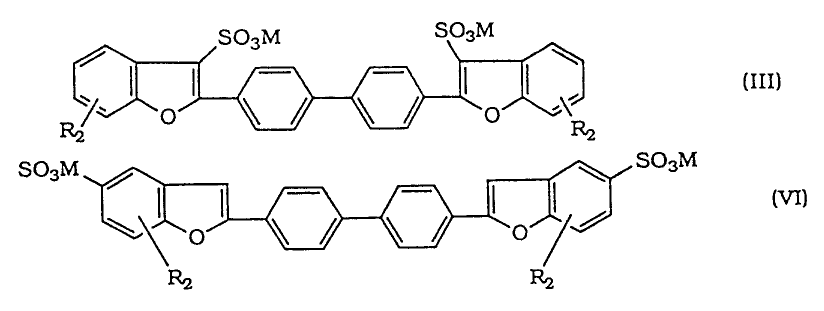
worin R₂ und M die oben angegebene Bedeutung haben. R₂ ist vorzugsweise Wasserstoff.

Ein weiterer Gegenstand dieser Anmeldung sind Verfahren zur Herstellung der Verbindungen der Formel (I), die dadurch gekennzeichnet sind, daß man
a) ein Mol der Verbindung der Formel (VII) 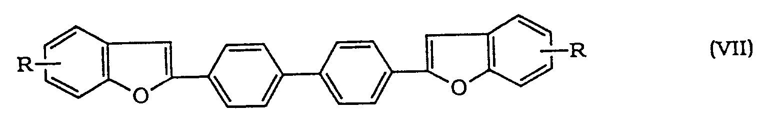 die gegebenenfalls mehrfach mit Resten R = Wasserstoff, C₁-C₄-Alkyl, C₁-C₄-Alkoxy, Halogen, Phenoxy und Benzyloxy substituiert ist, mit mindestens stöchiometrischen Mengen eines SO₃/Basen-Komplexes, in einem inerten organischen Lösungsmittel, bei Temperaturen von 20°C bis zum Siedepunkt des verwendeten Lösungsmittels umsetzt oder
b) ein Mol Bis-(halogenmethyl)-biphenyl mit mindestens 2 Mol Salicylaldehyd oder deren Anile der Formel (VIII) bzw. (IX) 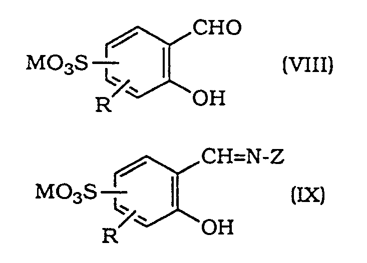 die gegebenenfalls mehrfach mit Resten R = Wasserstoff, C₁-C₄-Alkyl, C₁-C₄-Alkoxy, Halogen, Phenoxy und Benzyloxy substituiert sind und worin M = Wasserstoff und/oder ein Äquivalent eines nicht-chromophoren Kations und z = Phenyl oder Chlorphenyl bedeuten, verethert und die erhaltenen Bisphenylether der Formel (X) bzw. (XI) 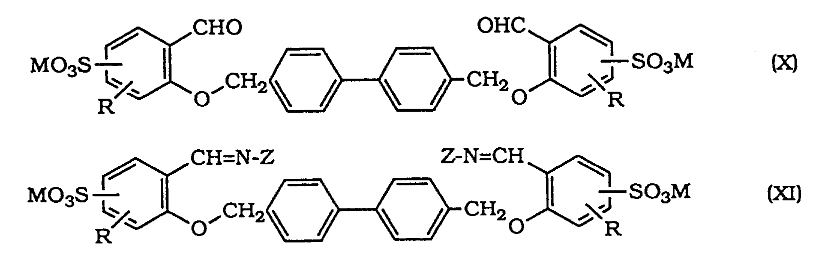 mit Basen cyclisiert.

Die Ausgangsverbindungen der Formel (VII), (VIII) und (IX) sind bekannt und können nach bekannten Methoden hergestellt werden. Die Zwischenprodukte der Formel (X) und (XI) sind neu und können isoliert werden. Vorteilhafterweise wird das Verfahren b) jedoch als Eintopfverfahren ohne Isolierung der Zwischenprodukte (X) und (XI) durchgeführt.

Im einzelnen werden nach dem Verfahren a) die Verbindungen der Formel (II) und (III) hergestellt.

Unter SO₃/Basen-Komplex sind Additionsverbindungen von SO₃ an organische Basen, z. B. Dioxan, vorzugsweise stickstoffhaltige Basen, wie zum Beispiel Triethylamin, N-Ethyldiisopropylamin, Dimethylformamid (DMF) und insbesondere Pyridin zu verstehen. Die Stabilität dieser Additionsverbindungen ist dabei entscheidend für den Sulfonierungsgrad. So werden bei Verwendung von 2 bis 6, insbesondere 3 bis 5 Mol SO₃/Pyridin-Komplex (bezogen auf den SO₃-Gehalt) pro Mol der Verbindung der Formel (VII) z.B. Verbindungen der Formel (II) und (III) hergestellt, SO₃/Basen-Komplexe sind bekannt und können nach bekannten Methoden hergestellt werden (E.E. Gilbert, E.P. Jones, Ind. Enging. Chem. 49, Nr. 9, Teil II, S. 1553 ff (1957); Beilstein 20, III/IV, 2232).

Beispiele für das beim Verfahren a) verwendete inerte organische Lösungsmittel sind gesättigte aliphatische Kohlenwasserstoffe wie Gasolin, Petrolether und Ligroin, halogenierte, aliphatische Kohlenwasserstoffe wie Chloroform, Tetrachlorkohlenstoff, Dichlorethan, Trichlorethan, Tetrachlorethan, Dichlorpropan, Trichlorpropan, Dichlordifluormethan und Dichlortetrafluorethan, Chlorbenzole wie Mono-, Di- und Trichlorbenzol, Nitrobenzole wie Nitrobenzol und Nitrotoluol sowie dicyclische Kohlenwasserstofe wie Cyclohexan, Methylcyclohexan und Dekalin.

Nach dem Verfahren b) werden die Verbindungen der Formel (IV) und insbesondere die Verbindungen der Formel (V) und (VI) hergestellt.

Die Veretherung erfolgt bei Temperaturen von 60° bis 140°C und insbesondere 100° bis 120°C in bekannter weise mittels einem Aequivalent einer Base, wie einem tert. Amin oder einer bei der folgenden Cyclisierung genannten Base, oder indem man die Verbindungen der Formel VIII oder IX bereits als Phenolat dieser Base einsetzt. Man arbeitet in einem polaren, aprotischen Lösungsmittel oder Lösungsmittelgemisch wie zum Beispiel Dimethylformamid, N-Methylpyrrolidon, Hexamethylphosphorsäuretriamid, Tetramethylharnstoff oder vorzugsweise Dimethylsulfoxid.

Die Cyclisierung erfolgt ebenfalls in einem polaren, aprotischen Lösungsmittel, vorzugsweise in demselben, in dem auch die Veretherung stattfindet, bei im Vergleich zur Veretherung leicht erhöhten Temperaturen und in Gegenwart einer Base, wie z.B. quaternären Ammoniumbasen, Erdalkalihydroxiden, Alkaliamiden, Alkalihydriden, Alkalicarbonaten, vorzugsweise aber Alkalialkoholaten wie Kalium-t-butylat und Natriummethylat sowie insbesondere Alkalihydroxiden wie Natrium-, Kalium- und Lithiumhydroxid. Die basischen Kondensationsmittel werden in mindestens stöchiometrischen Mengen, vorzugsweise im Ueberschuss, eingesetzt. Vorteilhaft arbeitet man unter Luftsauerstoffausschluss und Inertgasatmosphäre.

Die erfindungsgemässen Benzofuranylbiphenylverbindungen werden zum optischen Aufhellen von Textilien, z.B. Cellulose und/oder Polyamid enthaltende Gewebe sowie Papier, verwendet. Bevorzugt werden die erfindungsgemässen Benzofuranylbiphenylverbindungen in beispielsweise Hypochlorit enthaltende Bleichmittel oder insbesondere in Waschmittel eingearbeitet.

Die erfindungsgemässen Benzofuranylbiphenylverbindungen zeichnen sich durch ihre sehr gute Stabilität verbunden mit hervorragenden Aufhelleigenschaften aus.

Die nachfolgenden Beispiele erläutern die Erfindung; Teile bedeuten Gewichtsteile und Prozente Gewichtsprozente.

Beispiel 1: 11,8 g (0,03 Mol) der Verbindung der Formel (1) 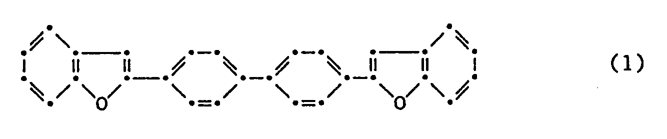
werden zusammen mit 21 g (0,12 Mol S0₃) Schwefeltrioxid Pyridin-Komplex (Gehalt ca. 45 % S0₃) im 50 ml Nitrobenzol unter Rühren während 15 Stunden auf 125°C erhitzt. Das Nitrobenzol wird dann mit Wasserdampf entfernt, die erhaltene Suspension mit Natronlauge alkalisch gestellt, das Produkt abgenutscht und aus einer Mischung von 150 ml Wasser und 150 ml Ethanol umkristallisiert. Man erhält so 4,7 g der Verbindung der Formel 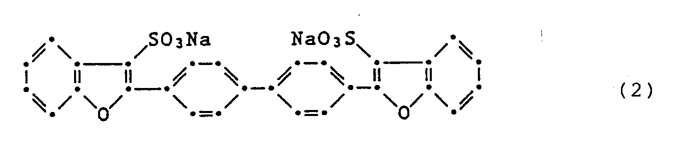
Beispiel 2: 27,1 g (0,11 Mol) Salicylaldehyd-5-sulfonsäuredinatriumsalz werden in 200 ml Dimethylsulfoxid bei 100°C verrührt. Zu der erhaltenen Suspension tropft man im Verlauf einer Stunde eine auf 60° vorgewärmte Lösung von 13,4 g (0,05 Mol) 4,4'-Bis(chlormethyl)-biphenyl 94%ig. Das Salicylaldehyd-derivat geht dabei in Lösung und es scheidet sich etwas Bis-benzylether der Formel 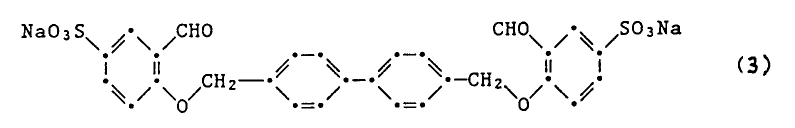
aus. Falls man diesen vollständig zu isolieren wünscht, dampft man das Lösungsmittel im Vakuum ab, verrührt den Rückstand mit 250 ml Wasser, filtriert und wäscht mit Wasser, Methanol und Aceton. Für die folgende Ringschlussreaktion ist die vorausgehende Isolierung der Verbindung der Formel (3) nicht notwendig.
Zu der vorgängig beschriebenen heissen Reaktionsmischung trägt man 8,2 g (0,2 Mol) fein pulverisiertes Natriumhydroxid unter Ueberleiten von Stickstoff ein, rührt weiter eine Stunde bei 100°C und 16 Stunden bei 120°C. Dabei fällt das Endprodukt teilweise aus. Man destilliert ca. 180 ml Lösungsmittel im Vakuum bei 120°C ab, lässt abkühlen und nutscht den Niederschlag ab. Nach wiederholtem Waschen mit Dimethylsulfoxid, Methanol und Wasser und Trocknen im Vakuum erhält man 13,1 g der Verbindung der Formel 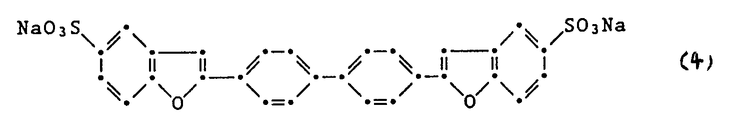
Beispiel 3: Eine Suspension von 11,8 g der Verbindung der Formel (4) in 240 ml Chlorbenzol, 10 ml Thionylchlorid und 0,3 ml Dimethylformamid wird 2 Stunden bei Rückflusstemperatur gerührt. Zur Beendigung der Reaktion fügt man noch etwas weiteres Thionylchlorid und Dimethylformamid hinzu und rührt weiter bei Rückflusstemperatur. Die erhaltene trübe Lösung wird heiss klärfiltriert, stark eingeengt und abgekühlt. Das ausgefallene Produkt wird abgenutscht, mit Chlorbenzol gewaschen und getrocknet. Man erhält das Disulfochlorid der Formel 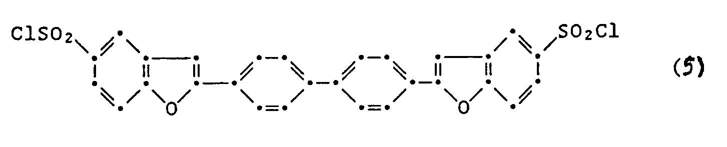
das aus Chlorbenzol umkristallisiert wird.

Eine Suspension von 3,7 g des Sulfochlorids der Formel (5) in 15 g Triethanolamin und 3 g Wasser wird eine Stunde bei 150° gerührt. Man entfernt das überschüssige Lösungsmittel weitgehend im Vakuum bei 120-150°C und versetzt den dickflüssigen Rückstand mit 50 ml Isopropanol. Das ausgefallene Produkt wird abgesaugt, wiederholt mit Isopropanol gewaschen und in ca. 40 ml Wasser aufgenommen. Die trübe Lösung wird mit 0,2 g Aktivkohle durch eine Drucknutsche klärfiltriert. Man erhält eine wässrige Lösung des Bis-triethanolamin-salzes der Formel 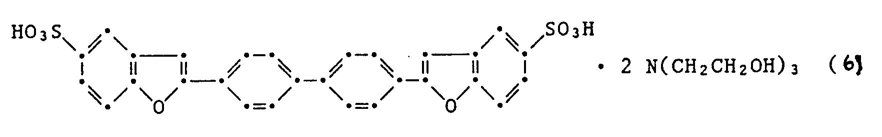
die noch Triethanolamin-hydrochlorid enthält.

Versetzt man eine solche Lösung bei 95°C unter Rühren mit überschüssiger konz. Salzsäure, so fällt das Mono-triethanolamin-salz der Formel 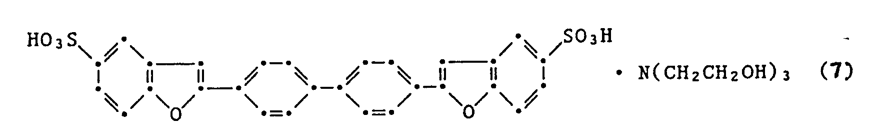
aus. Dieses wird abgenutscht, mit verd. Salzsäure und wenig Wasser gewaschen und getrocknet.

Beispiel 4: Ein Stück Polyamid 66 (Nylon Webtrikot) wird bei einem Flottenverhältnis von 1:20 in weichem Wasser, das 0,1 % der Verbindung der Formel (2) (Gewichtsprozent bezogen auf das Textilmaterial), sowie 3 g/l stabilisiertes Hydrosulfit und 1 ml/l Essigsäure 80%ig enthält, innerhalb von 30 Minuten von 40°C auf 98°C aufgeheizt, dann während 30 Minuten bei 98°C behandelt, und innerhalb von 15 Minuten wieder auf 40°C abgekühlt, kalt gespült und in einem Trockenschrank bei 60°C getrocknet.

Es resultiert ein hoher, brillanter Weissgrad. Nach üblicher Standard-Methode in einem ®Xenotest-Apparat belichtet, zeigt dieser Weisseffekt eine ausgezeichnete Lichtechtheit.

Beispiel 5: 1 g der Verbindung der Formel (2) wird in 1 l weichem Wasser, das 2 g/l Polyphosphat sowie 5 ml Essigsäure 80%ig enthält, gelöst. Ein Stück Polyamide 6 (®Perlon Webtrikot) wird bei einer Flottenaufnahme von 110 % mit diesem Bad kalt foulardiert, und anschliessend während 40 Sek. bei 190°C thermofixiert.
Nach dieser Behandlung weist das Textilmaterial einen hohen Weissgrad mit sehr guter Lichtechtheit auf.

Beispiel 6: Durch Sprühtrocknung eines Slurry bestehend aus 1 Teil Wasser und 1 Teil Waschmittel folgender Zusammensetzung
8,4 g lineares Dodecylbenzolsulfonat
3,1 g Talgalkohol-tetradecan-äthylenglykoläther (14 AeO)
3,7 g Na-Seife (vorwiegend aus Behen-Säure und C₁₄-C₂₀-Säuren)
45,8 g Na-Tripolyphosphat
7,9 g Na-Silikat
2,0 g Mg-Silikat
1,2 g Carboxymethylcellulose
0,2 g Aethylendiamin-tetraacetat
22,2 g Na-Sulfat
0,15 g der Verbindung (2)
wird ein Waschmittelgranulat mit einer Restfeuchte von ca. 5 % hergestellt.

4 g dieses Waschmittels werden in 1 l wasser (12° dH) bei einer Temperatur von 30°C aufgelöst. Fünf Stücke gebleichte Baumwolle à je 10 g werden in diesem Bad bei 30°C während 15 Minuten gewaschen, dann unter kaltem, fliessendem Wasser gespült und in einer Schwingmaschine bei einer Tourenzahl von ca. 1000 Touren/Minute während 30 Sekunden zentrifugiert. Dieser Waschprozess wird 3 mal durchgeführt, dann werden die Baumwoll-Stücke getrocknet und nach der Methode von Ganz mit einem Farbmessgerät (RFC 3 von Zeiss) auf ihren Weissgrad bestimmt.

Beispiel 7: Waschmittel werden gemäss Beispiel 6 hergestellt, die jedoch an Stelle der Verbindung (2) 0,1 g der Verbindung (4) oder (7) enthalten. Die 3-fache Wäsche wird wie im Beispiel 6 beschrieben durchgeführt, jedoch bei einer Temperatur von 90°C. Die erhaltenen Weissgrade liegen alle über 145.

## Patentansprüche

1. Benzofuranylbiphenylverbindungen der Formel (I) 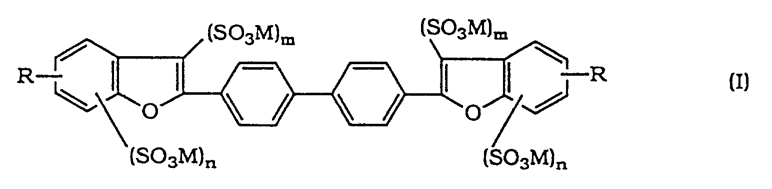 die gegebenenfalls mehrfach mit Resten R = Wasserstoff, C₁-C₄-Alkyl, C₁-C₄-Alkoxy, Halogen, Phenoxy und Benzyloxy substituiert sind und worin M = Wasserstoff und/oder ein Äquivalent eines nicht-chromophoren Kations und n eine Zahl Null oder 1 und m eine Zahl Null oder 1 bedeuten, mit der Bedingung, daß n und m nicht beide Null oder 1 sind.

2. Benzofuranylbiphenylverbindungen der Formel (II) gemäß Anspruch 1 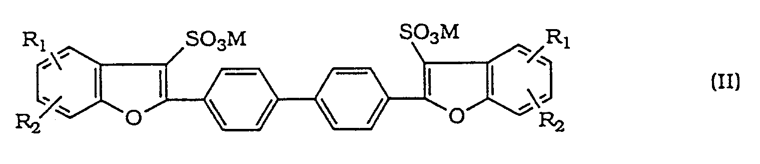 worin
R₁ = Wasserstoff, C₁-C₄-Alkyl, Chlor, C₁-C₄-Alkoxy, Phenoxy oder Benzyloxy, R₂ = Wasserstoff, C₁-C₄-Alkyl, Chlor oder C₁-C₄-Alkoxy und M = Wasserstoff und/oder ein Äquivalent eines nicht chromophoren Kations bedeuten.

3. Benzofuranylbiphenylverbindungen der Formel (III) gemäß Anspruch 2, 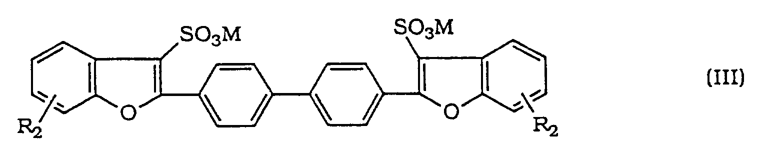 worin
R₂ = Wasserstoff, C₁-C₄-Alkyl, Chlor oder C₁-C₄-Alkoxy und M = Wasserstoff und/oder ein Äquivalent eines nicht-chromophoren Kations bedeuten.

4. Benzofuranylbiphenylverbindungen der Formel (IV) gemäß Anspruch 1 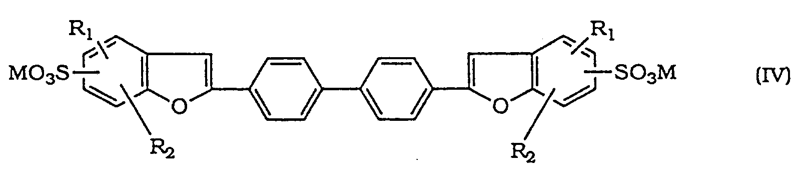 worin
R₁ = Wasserstoff, C₁-C₄-Alkyl, Chlor, C₁-C₄-Alkoxy, Phenoxy oder Benzyloxy, R₂ = Wasserstoff, C₁-C₄-Alkyl, Chlor oder C₁-C₄-Alkoxy und M = Wasserstoff und/oder ein Äquivalent eines nicht chromophoren Kations bedeuten.

5. Benzofuranylbiphenylverbindungen der Formel (V) gemäß Anspruch 4, 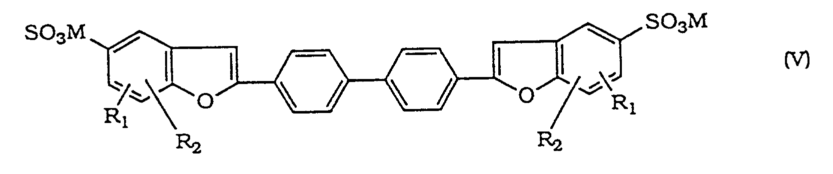 worin
R₁ = Wasserstoff, C₁-C₄-Alkyl, Chlor, C₁-C₄-Alkoxy, Phenoxy oder Benzyloxy, R₂ = Wasserstoff, C₁-C₄-Alkyl, Chlor oder C₁-C₄-Alkoxy und M = Wasserstoff und/oder ein Äquivalent eines nicht chromophoren Kations bedeuten.

6. Benzofuranylbiphenylverbindungen der Formel (VI) gemäß Anspruch 5. 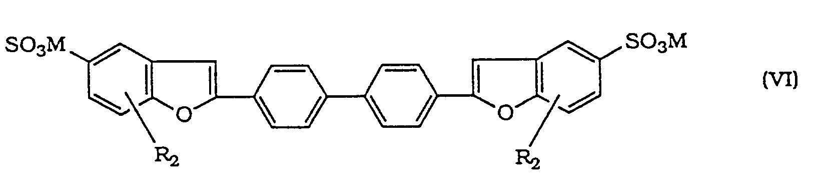 worin
R₂ = Wasserstoff, C₁-C₄-Alkyl, Chlor oder C₁-C₄-Alkoxy, M = Wasserstoff und/oder ein Äquivalent eines nicht-chromophoren Kations bedeuten.

7. Verfahren zur Herstellung der Verbindungen der Formel (I), **dadurch gekennzeichnet**, daß man
a) ein Mol der Verbindung der Formel (VII) 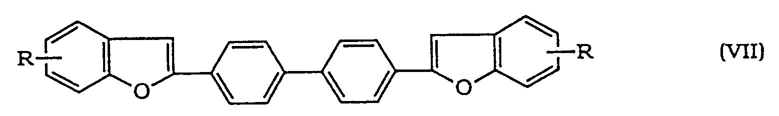 die gegebenenfalls mehrfach mit Resten R = Wasserstoff, C₁-C₄-Alkyl, C₁-C₄-Alkoxy, Halogen, Phenoxy und Benzyloxy substituiert ist, mit mindestens stöchiometrischen Mengen eines SO₃/Basen-Komplexes, in einem inerten organischen Lösungsmittel, bei Temperaturen von 20°C bis zum Siedepunkt des verwendeten Lösungsmittels umsetzt oder
b) ein Mol Bis-(halogenmethyl)-biphenyl mit mindestens 2 Mol Salicylaldehyd oder deren Anile der Formel (VIII) bzw. (IX) 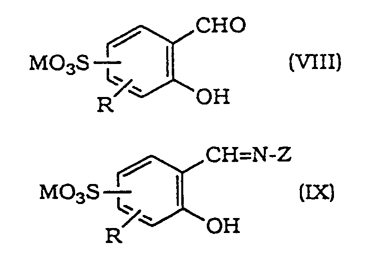 die gegebenenfalls mehrfach mit Resten R = Wasserstoff, C₁-C₄-Alkyl, C₁-C₄-Alkoxy, Halogen, Phenoxy und Benzyloxy substituiert sind und worin M = Wasserstoff und/oder ein Äquivalent eines nichtchromophoren Kations und Z = Phenyl oder Chlorphenyl bedeuten, verethert und die erhaltenen Bisphenylether der Formel (X) bzw. (XI) 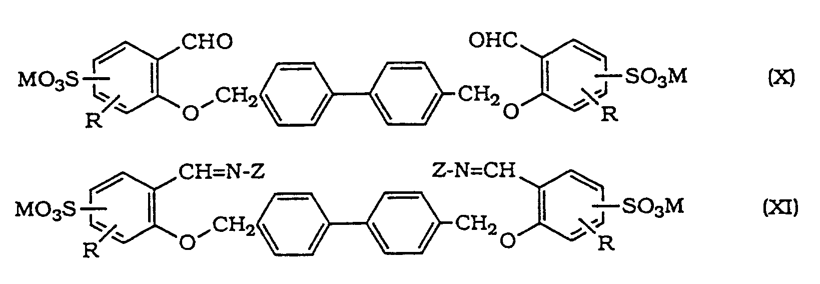 mit Basen cyclisiert.

8. Bisbenzylether der Formel 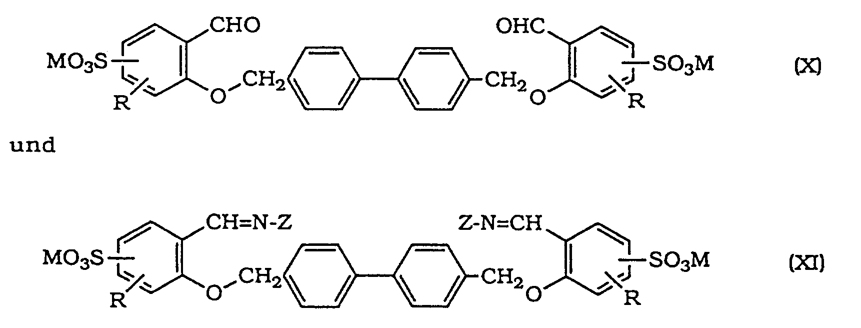 worin R, M, und Z die in Anspruch 7 angegebene Bedeutung haben.

9. Verwendung der in den Ansprüchen 1 bis 6 beanspruchten Verbindungen zum optischen Aufhellen.

10. Verwendung der Verbindungen gemäß Anspruch 9 in Hypochlorit enthaltenden Bleichmitteln sowie in Waschmitteln.

## Claims

1. A benzofuranylbiphenyl compound of the formula (I) 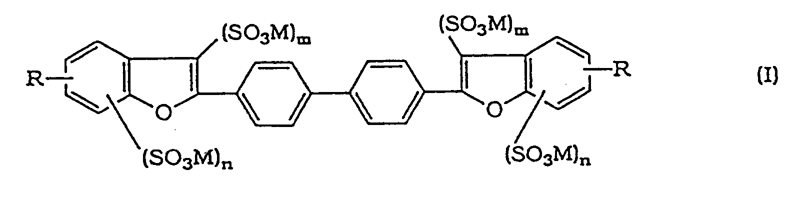 which is unsubstituted or polysubstituted by radicals R = hydrogen, C₁-C₄alkyl, C₁-C₄alkoxy, halogen, phenoxy and benzyloxy and in which M is hydrogen and/or one equivalent of a non-chromophoric cation and n is the number zero or 1 and m is the number zero or 1, subject to the condition that n and m are not both zero or 1.

2. A benzofuranylbiphenyl compound of the formula (II) according to claim 1 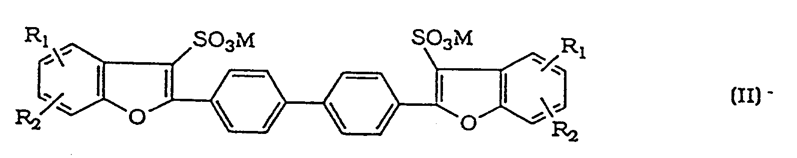 in which R₁ is hydrogen, C₁-C₄alkyl, chlorine, C₁-C₄alkoxy, phenoxy or benzyloxy, R₂ is hydrogen, C₁-C₄alkyl, chlorine or C₁-C₄alkoxy and M is hydrogen and/or one equivalent of a non-chromophoric cation.

3. A benzofuranylbiphenyl compound of the formula (III) according to claim 2 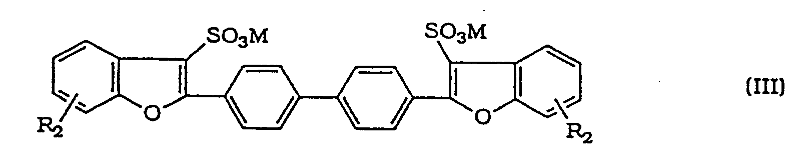 in which R₂ is hydrogen, C₁-C₄alkyl, chlorine or C₁-C₄alkoxy and M is hydrogen and/or one equivalent of a non-chromophoric cation.

4. A benzofuranylbiphenyl compound of the formula (IV) according to claim 1 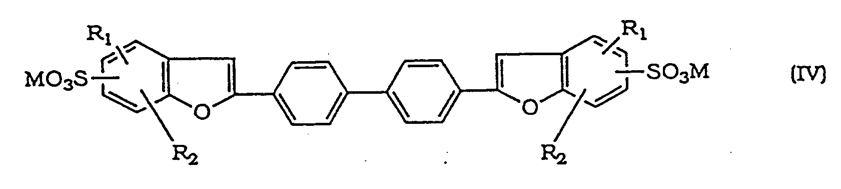 in which R₁ is hydrogen, C₁-C₄alkyl, chlorine, C₁-C₄alkoxy, phenoxy or benzyloxy, R₂ is hydrogen, C₁-C₄alkyl, chlorine or C₁-C₄alkoxy, M is hydrogen and/or one equivalent of a non-chromophoric cation.

5. A benzofuranylbiphenyl compound of the formula (V) according to claim 4 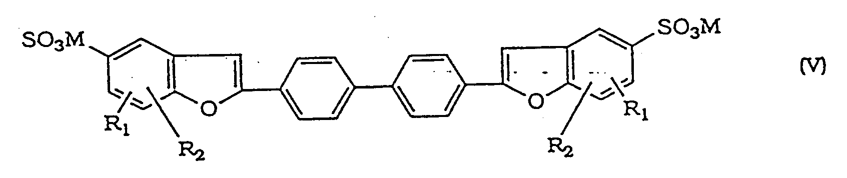 in which R₁ is hydrogen, C₁-C₄alkyl, chlorine, C₁-C₄alkoxy, phenoxy or benzyloxy, R₂ is hydrogen, C₁-C₄alkyl, chlorine or C₁-C₄alkoxy and M is hydrogen and/or one equivalent of a non-chromophoric cation.

6. A benzofuranylbiphenyl compound of the formula (VI) according to claim 5 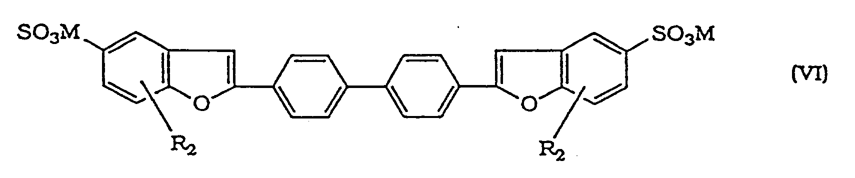 in which R₂ is hydrogen, C₁-C₄alkyl, chlorine or C₁-C₄alkoxy, and M is hydrogen and/or one equivalent of a non-chromophoric cation.

7. A process for the preparation of a compound of the formula (I), which comprises
a) reacting one mole of a compound of the formula (VII) 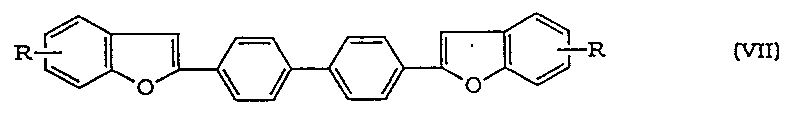 which is unsubstituted or polysubstituted by radicals R = hydrogen, C₁-C₄alkyl, C₁-C₄alkoxy, halogen, phenoxy and benzyloxy, with at least stoichiometric amounts of an SO₃/base complex in an inert organic solvent, at temperatures from 20°C up to the boiling point of the solvent used, or
b) etherifying one mole of bis(halomethyl)biphenyl with at least 2 moles of salicylaldehyde or anils thereof of the formula (VIII) or (IX) 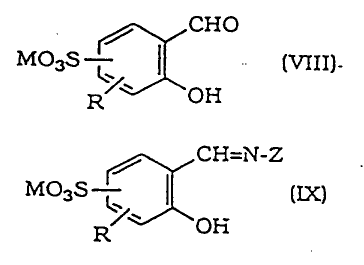 which are unsubstituted or polysubstituted by radicals R = hydrogen, C₁-C₄alkyl, C₁-C₄alkoxy, halogen, phenoxy and benzyloxy and in which M is hydrogen and/or one equivalent of a non-chromophoric cation and Z is phenyl or chlorophenyl, and cyclizing by means of bases the resulting bisphenyl ethers of the formula (X) or (XI)

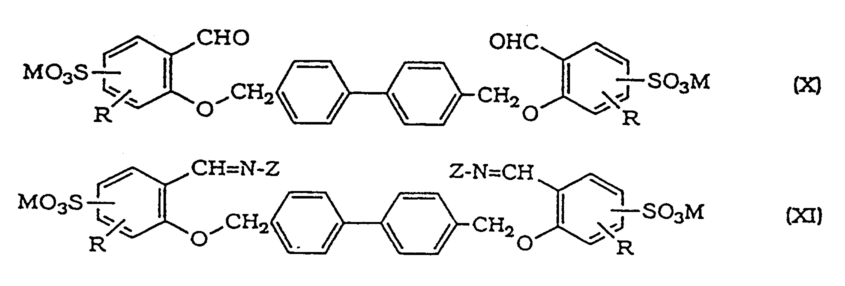

8. A bisbenzyl ether of the formula 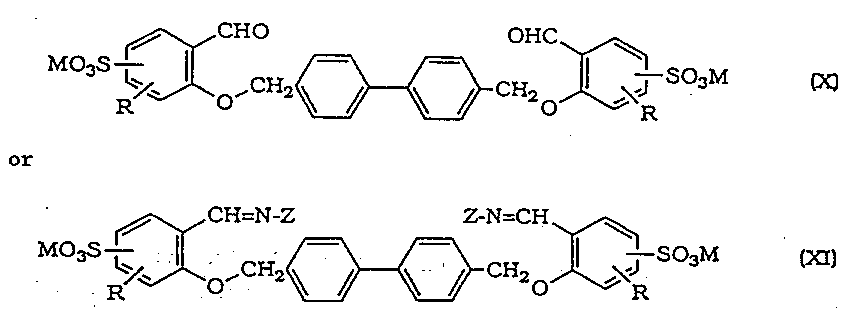 in which R, M and Z are as defined in claim 7.

9. The use of the compounds claimed in claims 1 to 6 for fluorescent whitening.

10. The use of the compounds according to claim 9 in bleaching agents containing hypochlorite and in detergents.

## Revendications

1. Dérivés de benzofuranylbiphényle de formule (I) : 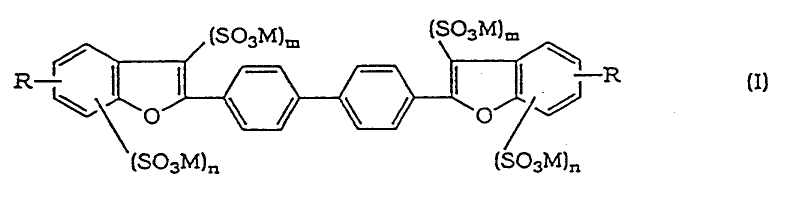 qui portent éventuellement plusieurs substituants représentés par les symboles R, qui représentent des atomes d'hydrogène ou d'halogène ou des groupes alkyle en C₁₋₄, alcoxy en C₁₋₄, phénoxy ou benzyloxy, M représentant un atome d'hydrogène et/ou un équivalent d'un cation non chromophore et n représentant un nombre valant zéro ou 1 et m représentant un nombre valant zéro ou 1, sous réserve que n et m ne représentent pas tous les deux ensemble zéro ou 1.

2. Dérivés de benzofuranylbiphényle conformes à la revendication 1, de formule (II) : 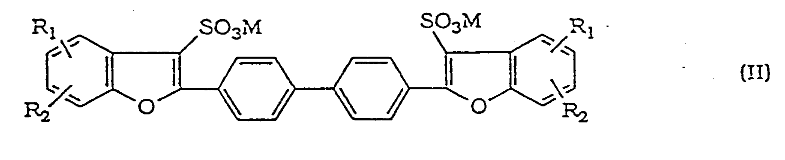 dans laquelle R₁ représente un atome d'hydrogène ou de chlore ou un groupe alkyle en C₁₋₄, alcoxy en C₁₋₄, phénoxy ou benzyloxy, R₂ représente un atome d'hydrogène ou de chlore ou un groupe alkyle en C₁₋₄ ou alcoxy en C₁₋₄, et M représente un atome d'hydrogène et/ou un équivalent d'un cation non chromophore.

3. Dérivés de benzofuranylbiphényle conformes à la revendication 2, de formule (III) : 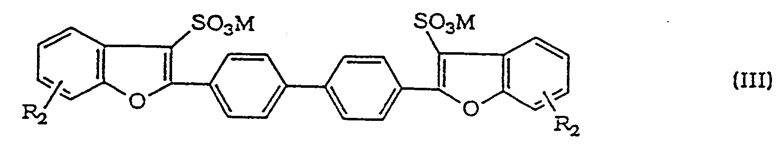 dans laquelle R₂ représente un atome d'hydrogène ou de chlore ou un groupe alkyle en C₁₋₄ ou alcoxy en C₁₋₄, et M représente un atome d'hydrogène et/ou un équivalent d'un cation non chromophore.

4. Dérivés de benzofuranylbiphényle conformes à la revendication 1, de formule (IV) : 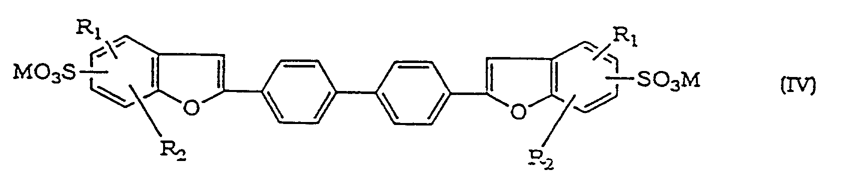 dans laquelle R₁ représente un atome d'hydrogène ou de chlore ou un groupe alkyle en C₁₋₄, alcoxy en C₁₋₄, phénoxy ou benzyloxy, R₂ représente un atome d'hydrogène ou de chlore ou un groupe alkyle en C₁₋₄ ou alcoxy en C₁₋₄, et M représente un atome d'hydrogène et/ou un équivalent d'un cation non chromophore.

5. Dérivés de benzofuranylbiphényle conformes à la revendication 4, de formule (V) : 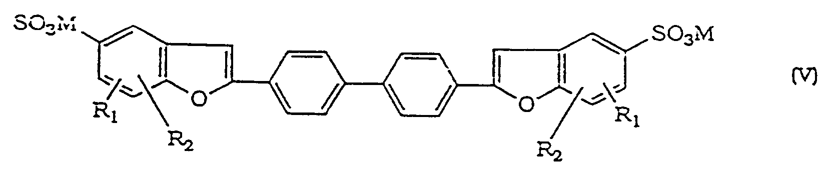 dans laquelle R₁ représente un atome d'hydrogène ou de chlore ou un groupe alkyle en C₁₋₄, alcoxy en C₁₋₄, phénoxy ou benzyloxy, R₂ représente un atome d'hydrogène ou de chlore ou un groupe alkyle en C₁₋₄ ou alcoxy en C₁₋₄, et M représente un atome d'hydrogène et/ou un équivalent d'un cation non chromophore.

6. Dérivés de benzofuranylbiphényle conformes à la revendication 5, de formule (VI) : 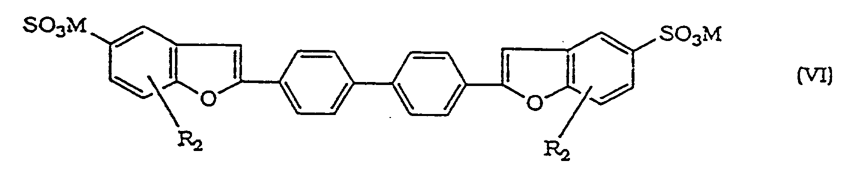 dans laquelle R₂ représente un atome d'hydrogène ou de chlore ou un groupe alkyle en C₁₋₄ ou alcoxy en C₁₋₄, et M représente un atome d'hydrogène et/ou un équivalent d'un cation non chromophore.

7. Procédé de préparation des composés de formule (I), caractérisé en ce que
a) on fait réagir une mole du composé de formule (VII) : 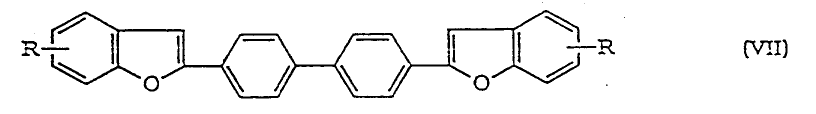 qui porte éventuellement plusieurs substituants représentés par les symboles R dont chacun représente un atome d'hydrogène ou d'halogène ou un groupe alkyle en C₁₋₄, alcoxy en C₁₋₄, phénoxy ou benzyloxy, avec une quantité au moins stoechiométrique d'un complexe d'une base et de SO₃, dans un solvant organique inerte, à des températures valant de 20°C au point d'ébullition du solvant utilisé, ou bien
b) on éthérifie une mole d'un bis(halogénométhyl)biphényle avec au moins 2 moles d'un salicylaldéhyde ou d'un anile de celui-ci, de formules respectives (VIII) et (IX) : 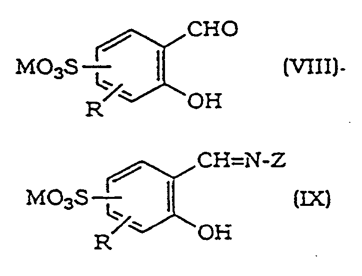 qui portent éventuellement plusieurs substituants représentés par les symboles R dont chacun représente un atome d'hydrogène ou d'halogène ou un groupe alkyle en C₁₋₄, alcoxy en C₁₋₄, phénoxy ou benzyloxy, M représentant un atome d'hydrogène et/ou un équivalent d'un cation non chromophore et Z représentant un groupe phényle ou chlorophényle,
et on effectue la cyclisation, à l'aide d'une base, des bis(phényléther) obtenus, de formules respectives (X) et (XI) :

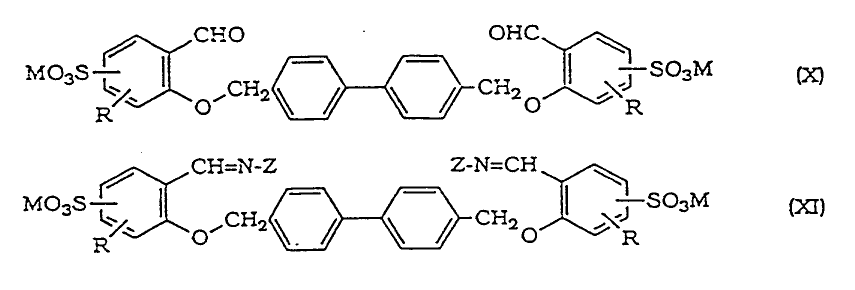

8. Bis(benzyléther) de formules : 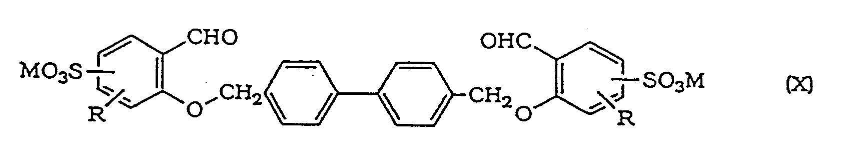 et 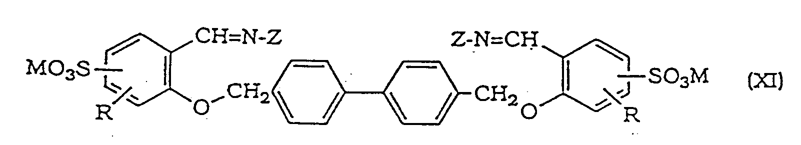 formules dans lesquelles R, M et Z ont les significations indiquées dans la revendication 7.

9. Utilisation des composés conformes aux revendications 1 à 6, à des fins d'azurage optique.

10. Utilisation de composés, conformes à la revendication 9, dans des agents de blanchiment contenant un hypochlorite ainsi que dans des détergents.
